# FASCICULE DE BREVET EUROPEEN

(11) **EP 1 157 980 B1**
(45) Date de publication et mention de la délivrance du brevet: **28.07.2004**
(21) Numéro de dépôt: 01401344.5
(22) Date de dépôt: 22.05.2001
(51) Int. Cl.: C07C 51/02, C07C 59/08

(54) **Procédé de préparation d'acide lactique par évapo-cristallisation**
Herstellung von Milchsäure durch Verdampfungs-Kristallisationsverfahren
Process for the production of lactic acid by evaporative crystallisation

(30) Priorité: 23.05.2000 FR 0006586
(43) Date de publication de la demande: 28.11.2001
(73) Titulaire: Roquette Frères, 62136 Lestrem (FR)
(72) Inventeur: Dubois, Eric, 62136 Lestrem (FR); Fouache, Catherine, 62113 Sailly/Labourse (FR)
(74) Mandataire: Boulinguiez, Didier

(56) Documents cités:
- EP-A- 0 393 818
- DE-C- 222 741
- GB-A- 907 321
- HONGO M ET AL: "NOVEL METHOD OF LACTIC ACID PRODUCTION BY ELECTRODIALYSIS FERMENTATION" APPLIED AND ENVIRONMENTAL MICROBIOLOGY,US,WASHINGTON,DC, vol. 52, no. 2, août 1986 (1986-08), pages 314-319, XP000852799 ISSN: 0099-2240

## Description

La présente invention est relative à un procédé particulier de préparation d'acide lactique à partir d'une solution aqueuse contenant de l'acide lactique sous forme de sel(s), notamment à partir d'un milieu de fermentation.

La présente invention concerne également un procédé de préparation d'acide lactique dont la pureté permet son utilisation non seulement dans des secteurs tels que les domaines d'applications alimentaires, les industries chimiques, agrochimiques, les matières plastiques, les cosmétiques mais surtout dans les domaines d'applications pharmaceutiques.

Selon l'invention, un « acide lactique de haute pureté » s'entend d'une qualité d'acide lactique satisfaisant aux normes pharmaceutiques de pureté (test de stabilité thermique de « The United States Pharmacopeia ») et de conformité par rapport aux normes du « Food Chemicals Codex ».

La préparation d'une solution aqueuse d'acide lactique sous forme de sel(s) s'entend habituellement de la fermentation de microorganismes des genres *Lactobacillus* (tels *L*. *acidophilus*, *L. delbrüeckii* ou *L*. *pentosus*), *Lactococcus*, *Enterococcus*, *Pediococcus*, *Vagococcus, Tetragenococcus*, *Aerococcus*, *Rhizopus*, *Bacillus* (tel *B. coagulans*)*, Screptococcus, Bifidobacterium ...*

En effet, il est connu que la croissance de la plupart de ces micro-organismes producteurs d'acide lactique, voire leur viabilité, est inhibée par la baisse du pH du milieu de fermentation, cette acidification forte du milieu étant provoquée par la production d'acides organiques, dont l'acide lactique lui-même.

Il est donc nécessaire de réguler le pH, et il est généralement admis qu'il faut le maintenir à une valeur comprise entre 4 et 7, de préférence supérieure à 4,5, par exemple comprise entre 5,5 et 6,5, par l'addition de bases, tels les hydroxydes de métaux alcalins ou alcalinoterreux, les carbonates ou les bicarbonates.

L'acide lactique se trouve donc dans ces milieux de fermentation sous forme de sels (lactates de sodium, de potassium, de calcium ou d'ammonium, seuls ou en mélange, en fonction de la base choisie pour réguler le pH du milieu de fermentation).

De ce fait, toutes les méthodes de récupération de l'acide lactique à partir de solutions aqueuses le contenant, et en particulier lorsque cette solution aqueuse est constituée d'un milieu de fermentation, doivent résoudre les mêmes difficultés, i.e. effectuer la séparation entre le ou les sel(s) de l'acide lactique, les microorganismes qui les ont produits et les impuretés du milieu de fermentation (sucres et protéines non consommés, et sels inorganiques de natures diverses), et de plus, effectuer la conversion des sels de l'acide lactique en acide lactique sous forme libre, ce qui nécessite également ensuite l'élimination de la base générée correspondante.

Diverses méthodes ont été proposées, pour la récupération de l'acide lactique à partir d'une solution aqueuse, et en particulier à partir d'un milieu de fermentation.

Toutes ces méthodes sont basées sur le même principe, i.e. l'extraction de l'acide lactique en tant que tel du milieu de fermentation.

Cependant, comme il a été précédemment montré, l'acide lactique est présent sous forme de sel (lactate).

Si la régulation du pH du milieu de fermentation est réalisée par des carbonates ou des bicarbonates de calcium, l'acide lactique sous forme libre peut être récupéré par exemple par acidification du milieu à l'acide sulfurique.

La réaction donne alors lieu à la formation de sulfates de calcium (gypse) qui précipitent et à la libération de l'acide lactique sous forme libre qui peut être ensuite par exemple adsorbé sur un support adapté et ensuite désorbé (GB-A-907 321).

Comme il est décrit dans EP 849.252, pour obtenir de l'acide lactique de haute pureté, il est généralement réalisé de multiples cristallisations, d'abord des lactates de calcium, pour éliminer les impuretés solubles du milieu de fermentation, et ensuite des sulfates de calcium libérés après traitement à l'acide sulfurique.

Ces étapes de cristallisation sont suivies de nombreuses étapes d'extraction complémentaires à l'éther ou par une amine à longue chaîne, combinées à des étapes de purification par chromatographie échangeuse d'ions, électrodialyse, et des réactions d'hydrolyse pour obtenir un acide lactique de haute pureté.

Le premier inconvénient de cette méthode par ailleurs efficace en termes de rendement, est la consommation élevée d'acide sulfurique, et surtout la production d'importantes quantités de gypse qui pose de sérieux problèmes en termes de traitement des déchets et de biodégrabilité pour l'environnement.

Le deuxième inconvénient est la complexité et le nombre élevé d'étapes nécessaires à l'obtention d'un acide lactique de haute pureté.

D'autres techniques ont donc été proposées, en conduisant la cristallisation des sels de l'acide lactique.

Par exemple, le brevet US 5.641.406 décrit, après l'étape de précipitation des lactates de calcium à l'acide sulfurique, et le traitement aux sels de ferrocyanure ou d'hexaferrocyanure pour éliminer les ions cuivre et fer, la décoloration au charbon actif de l'acide lactique « brut » ainsi obtenu, et après les étapes de purification subséquentes pour éliminer tous les sels résiduels, la concentration par évaporation, et donc la cristallisation des lactates.

ici encore, ce procédé souffre du nombre important d'étapes de purification et de la manipulation de produits chimiques toxiques.

Une solution à ces problèmes a été donnée dans US 5.210.296, par la mise en oeuvre d'un procédé consistant en :
- l'acidification d'une solution aqueuse contenant du lactate d'ammonium, en présence d'un alcool à 4 à 5 atomes de carbone utilisé comme diluant, avec de l'acide sulfurique (ou tout autre acide fort) en continu,
- l'élimination de l'eau du mélange acidifié par la distillation de l'azéotrope eau / alcool et de manière simultanée ou séquentielle, 1' élimination des cristaux de sulfates d'ammonium générés (ou des sels de l'acide fort générés),
- la distillation et l'hydrolyse de l'ester de l'acide lactique libéré pour produire un acide lactique libre d'une pureté de plus de 99,5 %.

Cependant, la difficulté de ce procédé réside notamment dans l'obligation d'éliminer les sulfates d'ammonium. Or, il est mentionné qu'il est impératif d'utiliser des alcools possédant de 4 à 5 atomes de carbone (en l'occurrence ici le n-butanol), pour obtenir des cristaux de sulfate d'ammonium suffisamment gros pour faciliter leur séparation par simple filtration du milieu réactionnel.

Il est alors possible de fabriquer grâce à cela un mélange azéotrope eau / n-butanol qu'il sera aisé d'éliminer par distillation en continu.

Il est donc encore obligatoire de passer par l'ester de l'acide lactique, sa distillation et son hydrolyse pour obtenir un acide lactique de pureté satisfaisante.

De ce fait, les procédés de l'état de la technique souffrent tous encore en pratique de cette succession d'étapes nombreuses et lourdes, qui rendent la purification de l'acide lactique à partir d'une solution aqueuse contenant de l'acide lactique sous forme des sel(s) particulièrement longue et fastidieuse.

De tout ce qui précède, il résulte qu'il existe un besoin non satisfait de disposer d'un procédé plus simple et moins coûteux, permettant la séparation, la concentration et la purification d'un acide lactique de haute pureté et ce, avec un excellent rendement à partir d'une solution aqueuse contenant de l'acide lactique sous forme de sel(s).

Soucieuse de développer un procédé qui permette de répondre mieux que ceux qui existent déjà aux contraintes de la pratique, la société Demanderesse a constaté que cet objectif pouvait être atteint par un procédé consistant à effectuer sur une solution aqueuse contenant de l'acide lactique sous forme des sel(s), une acidification de ladite solution aqueuse et une cristallisation dans des conditions particulières des sels de l'acide fort ainsi générés, ce qui permet d'obtenir directement un acide lactique libre de haute pureté.

La société Demanderesse a ainsi vaincu des préjugés techniques selon lesquels la mise en oeuvre d'une étape de cristallisation dans un protocole de purification d'acide lactique à partir d'une solution aqueuse contenant de l'acide lactique sous forme des sel(s):
- doit s'entendre de la cristallisation du sel de l'acide lactique pour l'extraire dudit milieu, et seulement ensuite de son acidification pour précipiter les sels de l'acide fort et générer l'acide lactique sous forme libre, ou
- s'il doit s'entendre de la cristallisation des sels de l'acide fort, ce doit être impérativement en milieu alcoolique, afin de générer des cristaux de caille suffisante pour permettre leur élimination dudit milieu, avec la formation simultanée de l'ester de l'acide lactique.

Le procédé de préparation d'acide lactique de haute pureté à partir d'une solution aqueuse contenant de l'acide lactique sous forme des sel(s) conforme à l'invention de la société Demanderesse est caractérisé par le fait que l'on :
- traite la solution aqueuse à l'aide d'un acide fort, de manière à libérer l'acide lactique sous forme libre et à générer les sels de l'acide fort correspondant,
- cristallise par évapo-cristallisation lesdits sels de l'acide fort,
- récupère l'acide lactique sous forme libre en solution.

La première étape du procédé conforme à l'invention consiste à traiter la solution aqueuse contenant l'acide lactique sous forme de sel(s) à l'aide d'un acide fort, de manière à libérer l'acide lactique sous forme libre et à générer les sels de l'acide fort correspondant.

Dans un mode préférentiel de réalisation du procédé conforme à l'invention, on utilise une solution aqueuse renfermant l'acide lactique sous forme de sels, obtenue à partir d'un milieu de fermentation d'un microorganisme producteur d'acide lactique. Ce milieu de fermentation est débarrassé desdits microorganismes par toute technique connue par ailleurs de l'homme du métier.

Les microorganismes producteurs d'acide lactique sont choisis indifféremment, et notamment dans le groupe constitué des *Lactobacillus*, *Lactococcus*, *Enterococcus,* *Pediococcus, Vagococcus, Tetragenococcus, Aerococcus, Rhizopus, Bacillus, Streptococcus* et *Bifidobacterium.* La composition de leur milieu de fermentation est largement décrite dans l'état de la technique.

L'ajustement du pH du milieu de fermentation à une valeur par exemple comprise entre 5 et 7 s'effectue en alimentant en continu le milieu de fermentation avec une base choisie de préférence dans le groupe constitué par NaOH, MgCO₂, Na₂CO₃, Ca(OH)₂, CaCO₃, KOH et NH₄OH, et est préférentiellement NH₄OH ou NaOH.

L'acide lactique est présent sous forme d'un sel de métal alcalino-terreux choisi dans le groupe constitué de l'ammonium, du magnésium, du calcium, du sodium et du potassium et est préférentiellement l'ammonium ou le sodium.

Selon l'invention, l'acide lactique contenu dans le milieu de fermentation se trouve donc partiellement ou totalement sous forme de sel(s) d'ammonium ou de sel(s) de sodium.

Quant à la séparation desdits microorganismes producteurs d'acide lactique, des autres composants du milieu de fermentation, elle peut consister en une microfiltration, une centrifugation ou une précipitation desdits microorganismes par des agents floculants. Ces techniques peuvent être également combinées.

Dans le procédé conforme à l'invention, on préfère une technique de microfiltration, qui utilise une membrane de microfiltration dont la porosité est adaptée à la taille des microorganismes considérés, par exemple des membranes TECHSEP de 0,1 µm de porosité pour des microorganismes producteurs d'acide lactique du genre *Lactobacillus.*

On procède ensuite avantageusement à une étape d'élimination des composants non ou peu ioniques du milieu de fermentation encore présents dans la solution aqueuse ainsi débarrassée des micro-organismes par une technique choisie dans le groupe des techniques de nanofiltration et/ou d'électrodialyse conventionnelle, comme il sera exemplifié ci-après.

Ces composants non ioniques s'entendent ici principalement des protéines et des sucres non complètement assimilés par les micro-organismes producteurs d'acide lactique.

L'étape d'électrodialyse conventionnelle consiste par exemple à traiter un milieu de fermentation débarrassé de sa biomasse, renfermant de 5 à 15 % en poids de lactate et coproduits de la fermentation, dans un module d'électrodialyse conventionnel afin de récupérer une solution enrichie en lactates et en sels, débarrassée des composés non ou peu ioniques.

La solution de lactate d'ammonium ou de sodium qui est alors récupérée en sortie d'électrodialyse conventionnelle présente par exemple une matière sèche comprise entre 4 et 15 % en poids, de préférence entre 5 à 10 % en poids.

Cette solution est ensuite avantageusement concentrée jusqu'à atteindre une matière sèche d'au moins 30 % en poids, de préférence entre 30 et 50 % en poids, par toute technique connue de l'homme du métier.

La solution de lactate d'ammonium ou de sodium ainsi concentrée est alors acidifiée à l'aide d'un acide fort. On choisit avantageusement de l'acide sulfurique à 98 %, et on amène le pH du milieu réactionnel à une valeur comprise entre 2 et 4, de préférence à une valeur de 2,5.

Cette réaction d'acidification conduit à séparer l'acide lactique sous forme libre et à générer les sels de l'acide fort correspondant, en l'occurrence ici les sulfates d'ammonium ou les sulfates de sodium.

La seconde étape du procédé conforme à l'invention consiste alors à cristalliser par évaporation desdits sels de l'acide fort, et plus particulièrement ici les sulfates d'ammonium ou de sodium.

Cette étape consiste à réaliser une évapocristallisation de la solution aqueuse contenant le mélange d'acide lactique libre et de sulfates d'ammonium ou de sulfates de sodium, de manière à permettre la cristallisation spécifique des sulfates d'ammonium ou de sodium, laissant l'acide lactique libre en solution dans la solution aqueuse mère.

La réaction est stoppée lorsque le système ne semble plus évaporer d'eau.

La masse cristalline ainsi obtenue peut être ensuite séparée de l'acide lactique libre en solution par une technique choisie dans le groupe des techniques de filtration et de centrifugation, et est préférentiellement la technique de centrifugation.

Les conditions d'évapocristallisation sont choisies de manière à obtenir une fraction constituée d'acide lactique libre à une matière sèche d'une valeur au moins égale à 70 % en poids, de préférence au moins égale à 90 % en poids, et une fraction renfermant au moins 90 % en poids de sulfate d'ammonium ou de sodium sous forme cristallisée, comme il sera exemplifié ci-après.

Il peut être enfin effectué une dernière étape consistant à éliminer les dernières impuretés présentes dans la fraction constituée d'acide lactique libre à une matière sèche ramenée à une valeur au moins égale à 10 % en poids, de préférence au moins égale à 25 % en poids. Lesdites impuretés sont composées principalement des ions, par exemple ammonium, que les différents traitements susmentionnés n'ont pas permis d'éliminer complètement ainsi que des sulfates résiduels.

Il est ainsi choisi avantageusement de :
a) déminéraliser la solution ainsi obtenue sur une résine cationique forte, puis une résine anionique faiblement basique,
b) concentrer l'acide lactique ainsi purifié.

La première étape consiste à éliminer l'ensemble des sels inorganiques par passage sur deux résines cationique forte puis anionique faible.

Dans la seconde étape, il est procédé à la concentration de la solution résultante de manière à l'amener à une matière sèche au moins égale à 90 % en poids.

De manière surprenante et inattendue, l'analyse de l'acide lactique ainsi séparé et purifié a révélé une qualité satisfaisant aux normes pharmaceutiques de pureté (conformément à « The United States Pharmacopeia ») et de conformité par rapport aux normes du « Food Chemicals Codex ».

D'autres caractéristiques et avantages de l'invention apparaîtront à la lecture des exemples qui suivent. Ils ne sont toutefois donnés ici qu'à titre illustratif et non limitatif.

### EXEMPLE 1

On part d'un milieu de fermentation de *Lactococcus* contenant 82 g/l d'acide lactique sous forme de lactates d'ammonium, 0,8 % de protéines et 0,5 % de sucres résiduels, débarrassé de ses micro-organismes par microfiltration.

650 l de cette solution alimentent un module d'électrodialyse type EUR6B EURODIA, équipé de membranes échangeuses d'ions (NEOSEPTA-TOKUYAMA SODA) de type cationiques CMX-S et anioniques AMX Sb de 5,6 m² de surface active, en suivant les paramètres de fonctionnement définis par le constructeur, à 35°C, de manière à obtenir un taux de récupération d'acide lactique sous forme de lactates d'ammonium de 98 %.

La solution ainsi électrodialysée permet de récupérer dans la fraction enrichie en lactates d'ammonium 90% d'acide lactique sous sa forme sel.

Cette solution est concentrée par évaporation sous vide à une pression de 0,9 bar et à une température de 60°C sur évaporateur à flot tombant de type WIEGAND.

La solution concentrée ainsi obtenue (120 l à 40 % en poids sur sec) est acidifiée par H₂SO₄ à 98 % jusqu'à un pH de 2,5.

Cette solution acidifiée subit alors une étape d'évapocristallisation consistant à soutirer sous vide l'eau résiduelle sous agitation, à une pression fixée à 0,8 bar à une température de 60°C.

L'évapocristallisation est conduite pendant 10 h.

En fin d'évapocristallisation, on obtient 52 kg d'une masse cristallisée renfermant 36 % en poids de cristaux individualisés de sulfates d'ammonium.

Une étape de centrifugation en centrifugeuse horizontale type ROUSSELET permet de séparer les cristaux de sulfates d'ammonium de la solution lactique sous forme libre purifiée.

Ladite solution d'acide lactique est récupérée à une matière sèche de 95 % pour une richesse de 95 % en acide lactique.

Ladite solution est ensuite ramenée à 25 % en poids de matière sèche et déminéralisée sur résines cationiques fortes et anioniques faibles, par toute méthode connue en soi par l'homme du métier.

L'acide lactique ainsi déminéralisé est concentré par évaporation de manière à l'amener à une manière sèche de 90 % en poids.

L'acide lactique ainsi séparé et purifié a révélé une qualité satisfaisant aux normes pharmaceutiques de pureté (conformément à « The United states Pharmacopeia ») et de conformité par rapport aux normes du « Food Chemicals Codex ». Plus particulièrement, l'analyse a révélé une teneur en acide L lactique de 97,5 %, une teneur en cendres inférieure à 0,1 %. De plus, les tests de « substance aisément carbonisable », « acides organiques » et « teneur en sucres réducteurs » sont conformes aux spécifications requises.

### EXEMPLE 2

On procède de la même manière que décrit dans l'exemple 1, en partant d'un milieu de fermentation de *Lactococcus* contenant 80 g/l d'acide lactique sous forme de lactates de sodium, 0,9 % de protéines et 0,4 % de sucres résiduels, débarrassé de ses micro-organismes par microfiltration.

207 l de cette solution alimentent un module d'électrodialyse type EUR6B EURODIA, équipé de membranes échangeuses d'ions (NEOSEPTA-TOKUYAMA SODA) de type cationiques CMX-S et anioniques AMX Sb de 5,6 m² de surface active, en suivant les paramètres de fonctionnement définis par le constructeur, à 35°C, de manière à obtenir un taux de récupération d'acide lactique sous forme de lactates de sodium de 97 %.

La solution ainsi électrodialysée permet de récupérer dans la fraction enrichie en lactates de sodium 90 % d'acide lactique sous sa forme sel.

Cette solution est concentrée par évaporation sous vide à une pression de 0,9 bar et à une température de 60°C sur évaporateur à flot tombant de type WIEGAND.

La solution concentrée ainsi obtenue (41,5 l à 40 % en poids sur sec) est acidifiée par H₂SO₄ à 98 % jusqu'à un pH de 2,5.

Cette solution acidifiée subit alors une étape d'évapocristallisation consistant à soutirer sous vide l'eau résiduelle sous agitation, à une pression fixée à 0,8 bar à une température de 60°C.

L'évapocristallisation est conduite pendant 10 h.

En fin d'évapocristallisation, on obtient 19 kg d'une masse cristallisée renfermant 40 % en poids de cristaux individualisés de sulfates de sodium.

Une étape de centrifugation en centrifugeuse horizontale type ROUSSELET permet de séparer les cristaux de sulfates de sodium de la solution lactique sous forme libre purifiée.

Ladite solution d'acide lactique est récupérée à une matière sèche de 95 % pour une richesse de 95 % en acide lactique.

Ladite solution est ensuite ramenée à 25 % en poids de matière sèche et déminéralisée sur résines cationiques fortes et anioniques faibles, par toute méthode connue en soi par l'homme du métier.

L'acide lactique ainsi déminéralisé est concentré par évaporation de manière à l'amener à une matière sèche de 90 % en poids.

L'acide lactique ainsi séparé et purifié a révélé une qualité satisfaisant aux normes pharmaceutiques de pureté (conformément à « The United States Pharmacopeia ») et de conformité par rapport aux normes du « Food Chemicals Codex ». Plus particulièrement, l'analyse a révélé une teneur en acide L-lactique de 98 %, une teneur en cendres inférieure à 0,1%. De plus, les tests de « substance aisément carbonisable », « acides organiques » et « teneur en sucres réducteurs » sont conformes aux spécifications requises.

## Revendications

1. Procédé de préparation d'acide lactique de haute pureté à partir d'une solution aqueuse contenant ledit acide sous forme de sel(s), **caractérisé par le fait que** l'on :
- traite la solution aqueuse à l'aide d'un acide fort, de manière à libérer l'acide lactique sous forme libre et à générer les sels de l'acide fort correspondant,
- cristallise par évapo-cristallisation lesdits sels de l'acide fort,
- récupère l'acide lactique sous forme libre en solution.

2. Procédé selon la revendication 1, **caractérisé par le fait que** la solution aqueuse contenant l'acide lactique sous forme de sel(s) est un milieu de fermentation débarrassé de ses micro-organismes.

3. Procédé selon la revendication 2, **caractérisé par le fait que** le milieu de fermentation est soumis à une étape d'électrodialyse de manière à obtenir une solution aqueuse électrodialysée débarrassée des composants non ioniques du milieu de fermentation.

4. Procédé selon la revendication 3, **caractérisé par le fait que** la solution aqueuse électrodialysée est concentrée à une matière sèche d'au moins 30%.

5. Procédé selon la revendication 4, **caractérisé par le fait que** la solution aqueuse électrodialysée est concentrée à une matière sèche comprise entre 30 et 50 %.

6. Procédé selon l'une quelconque des revendications 1 à 5, **caractérisé par le fait que** l'acide fort est de l'acide sulfurique à 98 %.

7. Procédé selon l'une quelconque des revendications 1 à 6, **caractérisé par le fait que** les cristaux des sels de l'acide fort sont séparés de l'acide lactique sous forme libre par une technique choisie dans le groupe des techniques de filtration et de centrifugation.

8. Procédé selon la revendication 7, **caractérisé par le fait que** les cristaux des sels de l'acide fort sont séparés de l'acide lactique sous forme libre par la technique de centrifugation.

9. Procédé selon l'une quelconque des revendications 1 à 8, **caractérisé par le fait que** l'acide lactique est présent sous forme d'un sel de métal alcalino-terreux choisi dans le groupe constitué de l'ammonium, du magnésium, du calcium, du sodium et du potassium.

10. Procédé selon la revendication 9, **caractérisé par le fait que** l'acide lactique est présent sous forme d'un sel de métal alcalino-terreux qui est l'ammonium ou le sodium.

## Patentansprüche

1. Verfahren zur Herstellung einer Milchsäure hoher Reinheit aus einer wässerigen Lösung enthaltend die Säure in Form eines Salzes (in Form von Salzen), **dadurch gekennzeichnet, dass**
- die wässerige Lösung mit Hilfe einer starken Säure behandelt wird, um die Milchsäure in freier Form freizusetzen und die entsprechenden Salze der starken Säure zu bilden,
- die Salze der starken Säure durch Verdampfungskristallisation kristallisiert werden,
- die Milchsäure in freier Form in Lösung erhalten wird.

2. Verfahren nach Anspruch 1, **dadurch gekennzeichnet, dass** die wässerige Lösung enthaltend die Milchsäure in Form eines Salzes (in Form von Salzen) ein Fermentationsmedium ist, das von seinen Mikroorganismen befreit ist.

3. Verfahren nach Anspruch 2, **dadurch gekennzeichnet, dass** das Fermentationsmedium einem elektrodialytischen Schritt unterzogen wird, um eine wässerige elektrodialysierte Lösung zu erhalten, die von nichtionischen Bestandteilen des Fermentationsmediums befreit ist.

4. Verfahren nach Anspruch 3, **dadurch gekennzeichnet, dass** die wässerige elektrodialysierte Lösung auf eine Trockenmasse von wenigsten 30 % konzentriert wird.

5. Verfahren nach Anspruch 4, **dadurch gekennzeichnet, dass** die wässerige elektrodialysierte Lösung auf eine Trockenmasse umfasst zwischen 30 und 50 % konzentriert wird.

6. Verfahren nach einem der Ansprüche 1 bis 5, **dadurch gekennzeichnet, dass** die starke Säure 98%ige Schwefelsäure ist.

7. Verfahren nach einem der Ansprüche 1 bis 6, **dadurch gekennzeichnet, dass** die Salzkristalle der starken Säure von der Milchsäure in freier Form durch eine Technik getrennt wird, die aus der Gruppe von Filtrations- und Zentrifugationstechniken ausgewählt wird.

8. Verfahren nach Anspruch 7, **dadurch gekennzeichnet, dass** die Salzkristalle der starken Säure von der Milchsäure in freier Form durch die Zentrifugationstechnik getrennt wird.

9. Verfahren nach einem der Ansprüche 1 bis 8, **dadurch gekennzeichnet, dass** die Milchsäure in Form eines Erdalkalimetallsalzes vorhanden ist, das aus der Gruppe gebildet aus Ammonium, Magnesium, Calcium, Natrium und Kalium ausgewählt wird.

10. Verfahren nach Anspruch 9, **dadurch gekennzeichnet, dass** die Milchsäure in Form eines Erdalkalimetallsalzes vorhanden ist, das Ammonium oder Natrium ist.

## Claims

1. A process for the preparation of high purity lactic acid from an aqueous solution containing said acid in the form of salt(s), **characterized by** the fact that:
- the aqueous solution is treated with a strong acid, in order to liberate lactic acid in the free form and to produce corresponding salts of the strong acid,
- said salts of the strong acid are crystallised by evaporative crystallisation,
- lactic acid is recovered in the free form in solution.

2. A process according to claim 1, **characterized by** the fact that the aqueous solution containing lactic acid in the form of salt(s) is a fermentation medium from which its microorganisms have been removed.

3. A process according to claim 2, **characterized by** the fact that the fermentation medium undergoes an electrodialysis step in order to obtain an electrodialysed aqueous solution from which the nonionic constituents of the fermentation medium have been removed.

4. A process according to claim 3, **characterized by** the fact that the electrodialysed aqueous solution is concentrated to a dry matter content of at least 30 %.

5. A process according to claim 4, **characterized by** the fact that the electrodialysed aqueous solution is concentrated to a dry matter content in the range from 30 % to 50%.

6. A process according to one of the claims 1 to 5, **characterized by** the fact that in the strong acid is 98 % sulphuric acid.

7. A process according to one of the claims 1 to 6, **characterized by** the fact that the crystals of salts of the strong acid are separated from lactic acid in the free form by a method selected from the group of filtration and centrifugation methods.

8. A process according to claim 7, **characterized by** the fact that the crystals of salts of the strong acid are separated from lactic acid in the free form by the centrifugation method.

9. A process according to any one of the claims 1 to 8, **characterized by** the fact that lactic acid is present in the form of a salt of alkaline earth metal selected from the group consisting of ammonium, magnesium, calcium, sodium and potassium.

10. A process according to claim 9, **characterized by** the fact that the lactic acid is present in the form of a salt of alkaline earth which is ammonium or sodium.
